# EUROPÄISCHE PATENTSCHRIFT

(11) **EP 4 463 063 B1**
(45) Veröffentlichungstag und Bekanntmachung des Hinweises auf die Patenterteilung: **03.06.2026**
(21) Anmeldenummer: 23702519.2
(22) Anmeldetag: 09.01.2023
(51) Int. Cl.: A61B 5/07

(54) **SCHLUCKBARER ELEKTROCHEMISCHER SENSOR**
INGESTIBLE ELECTROCHEMICAL SENSOR
CAPTEUR ÉLECTROCHIMIQUE INGÉRABLE

(30) Priorität: 11.01.2022 DE 102022100522
(43) Veröffentlichungstag der Anmeldung: 20.11.2024
(73) Patentinhaber: Drägerwerk AG & Co. KGaA, 23558 Lübeck (DE)
(72) Erfinder: NAUBER, Andreas, 23558 Lübeck (DE); SICK, Michael, 23558 Lübeck (DE); MATTERN-FRÜHWALD, Marie-Isabell, 23558 Lübeck (DE); DENNIER, Silja, 23558 Lübeck (DE)
(86) Internationale Anmeldenummer: PCT/DE2023/100008
(87) Internationale Veröffentlichungsnummer: WO 2023/134827

(56) Entgegenhaltungen:
- DE-A1- 102013 101 735
- US-A1- 2010 185 055
- US-A1- 2013 289 368
- US-A1- 2020 182 823

## Beschreibung

Die Erfindung betrifft einen Elektrochemischen Sensor, der sich dadurch auszeichnet, dass er schluckbar ist und zum Nachweis von Gasen im Intestinaltrakt eines Menschen verwendbar ist.

Elektrochemische Sensoren sind grundsätzlich bekannt. Dabei handelt es sich um elektrochemische Zellen, die wenigstens eine Arbeitselektrode und eine Gegenelektrode aufweisen, wobei zwischen den Elektroden mit Hilfe einer leitfähigen Flüssigkeit, dem sogenannten Elektrolyten, ein Stromfluss zwischen den Elektroden stattfinden kann, sofern gewisse Analyten in den Bereich der Elektroden gelangen. Bei den Analyten handelt es sich üblicherweise um Gase.

Nachteilhaft an üblichen Elektrochemischen Sensoren ist jedoch, dass die Materialien, die üblicherweise für die elektrochemische Reaktion benötigt werden, also sowohl Elektrolyt als auch Elektroden, häufig toxisch oder zumindest schädlich für Organismen sind.

Der Einsatzbereich von herkömmlichen Elektrochemischen Sensoren ist insofern begrenzt.

Dennoch ist es aus medizinischer Sicht von Interesse, gewisse Gase innerhalb eines Organismus nachweisen zu können. So kann es beispielsweise im Zusammen hang mit entzündlichen Darmerkrankungen von Interesse sein, die Entwicklung von H₂S- oder NO-Gas zu überwachen. Beispielsweise könnte anhand der Entwicklung dieser Gase der Schweregrad einer entsprechenden Krankheit, die oft in Wellen verläuft, vorhergesagt werden.

Verschluckbare Sensoren sind beispielsweise aus US 10,326,139 B2 bekannt. Dabei handelt es sich jedoch zumeist entweder um optische Sensoren oder um pH-Sensoren. Diese sind nur begrenzt zum Nachweis von Gasen geeignet. Zwar können optische Sensoren verwendet werden, um Methan in großen Konzentrationen nachzuweisen, häufiger ist jedoch ihr Einsatze zur Bildgebung des Magen-Darm-Traktes. pH-Sensoren werden hingegen zumeist zur Überprüfung des pH-Wertes des Magens verwendet.

Aus Yoshida, S., Miyaguchi, H. & Nakamura, T. (2018). Proof of Concept for Tablet-Shaped Ingestible Core-Body Thermometer with Gastric Acid Battery. IEEE Sensors Journal; 18(23), 9755-9762; doi: 10.1109/JSEN.2018.2871064 ist außerdem beispielsweise eine Batterie bekannt, die zum Betrieb einer verschluckbaren Elektronik verwendet werden kann. Auch bei einer Batterie handelt es sich um eine elektrochemische Zelle. In diesem Fall liegen allerdings die Elektroden in Form von Metallplättchen frei an der Oberfläche. Die Wirkung als Batterie entsteht, sobald diese frei an der Oberfläche liegenden Elektroden mit Magensaft aus der Umgebung in Kontakt kommen. Es sei angemerkt, dass es sich hier zwar um eine elektrochemische Zelle mit Magensaft als Elektrolyt handelt, die allgemeine Funktionsweise unterscheidet sich zwischen Batterien und Elektrochemischen Gassensoren grundlegend. Die von Yoshida, S., Miyaguchi, H. & Nakamura, T. (2018) vorgeschlagene Batterie ist insofern auf keinen Fall für den Nachweis von Gasen geeignet.

Aus Dokument US2013/289368 A1 ist ein elektrochemischer Sensor gemäß dem Oberbegriff des Anspruchs 1 bekannt. Ein weiterer elektrochemischer Sensor ist im Dokument US2020/182823 A1 offenbart.

Aufgabe der vorliegenden Erfindung ist es daher, einen elektrochemischen Gassensor bereitzustellen, der zum Nachweis von Gasen im Verdauungstrakt eines Individuums verwendbar ist.

Hauptmerkmale der Erfindung sind im kennzeichnenden Teil von Anspruch 1 angegeben. Ausgestaltungen sind Gegenstand der Ansprüche 2 bis 14.

Es wird ein elektrochemischer Gassensor mit einem Gehäuse, mit wenigstens einer Arbeitselektrode und mit einer Gegenelektrode vorgeschlagen, wobei das Gehäuse einen Gaseinlass hat, der mit einer gasdurchlässigen Membran verschlossen ist. Der elektrochemische Gassensor ist dadurch gekennzeichnet, dass das Gehäuse wenigstens einen Elektrolyteinlass hat, der mit einem hydrophilen Dichtstoff ausgefüllt ist. Der elektrochemische Gassensor ist bevorzugt ein schluckbarer Gassensor zum Nachweis von Gasen im Intestinaltrakt eines Menschen.

Ein solcher Gassensor kann von einem zu untersuchenden Individuum verschluckt werden, so dass er in den Gastrointestinaltrakt gelangt. Mit anderen Worten es ist eine schluckbare Sensorvorrichtung. Im Gastrointestinaltrakt, bevorzugt im Magen, kann dann der hydrophile Dichtstoff dafür sorgen, dass Magensaft durch den Elektrolyteinlass in das Innere des Gehäuses gelangt, bevorzugt in den nachfolgend beschriebenen Innenraum. Dazu ist es vorteilhaft, wenn der hydrophile Dichtstoff durchlässig für wässrige Flüssigkeiten ist, wie z.B. Magensaft, aber dichtend gegenüber Partikeln, beispielsweise gegenüber Speisepartikeln. Der Magensaft kann auf diese Weise in Kontakt mit den Elektroden, insbesondere der Arbeitselektrode und der Gegenelektrode, gelangen und in der Folge im Inneren des Gassensors als Elektrolyt dienen. Durch das Einströmen des Magensaftes durch den Elektrolyteinlass entsteht nach dem Verschlucken der erfindungsgemäßen Vorrichtung im Körperinneren des Individuums mithin ein voll funktionsfähiger elektrochemischer Gassensor. Mit anderen Worten, die erfindungsgemäße Vorrichtung kann auch als schluckbarer, elektrochemischer Instantgassensor bezeichnet werden. Dies bietet den großen Vorteil, dass mit Hilfe der erfindungsgemäßen Vorrichtung beim Betrieb des elektrochemischen Gassensors innerhalb des Körpers eines zu untersuchenden Individuums auf die Verwendung herkömmlicher Elektrolyte, insbesondere toxischer und/oder gesundheitsschädlicher Elektrolyte, verzichtet werden kann. Dies ist ein großer Vorteil der Erfindung.

In jedem Fall ist das Gehäuse ist ein Hohlkörper mit einer Außenseite und einem Innenraum. Im Innenraum sind die Komponenten der Elektrochemischen Zelle angeordnet. Im betriebsbereiten Zustand ist außerdem der als Elektrolyt dienende Magensaft im Innenraum angeordnet.

Günstig ist es dabei, wenn das Gehäuse auch einen vom Elektrolyteinlass verschiedenen Gaseinlass aufweist. Durch diesen Gaseinlass kann dann nachzuweisendes Gas in die elektrochemische Zelle gelangen und anschließend an der Arbeitselektrode umgesetzt werden.

Man erkennt, dass es insofern vorteilhaft ist, wenn das Gehäuse mehrere Öffnungen hat, nämlich wenigstens einen Gaseinlass und wenigstens einen Elektrolyteinlass. Der Gaseinlass ist ein Loch in der Wand des Gehäuses durch das Gas in den Innenraum des Gehäuses eindringen kann. Um das Eindringen von Flüssigkeit durch den Gaseinlass zu verhindern ist es sinnvoll, wenn der Gaseinlass mit einer Membran verschlossen ist, die zwar gasdurchlässig ist, gegenüber Flüssigkeit und Partikeln jedoch eine Barriere bildet. Auch der Elektrolyteinlass ist ein Loch in der Wand des Gehäuses.

Im Innenraum kann das Gas dann in Kontakt mit der Arbeitselektrode und dem durch den Elektrolyteinlass eingeströmten Elektrolyten kommen, wodurch es in der Folge zur elektrochemischen Reaktion kommt. Durch die elektrochemische Reaktion kommt es zu einem Stromfluss zwischen Arbeits- und Gegenelektrode. Dieser Stromfluss kann dann detektiert werden.

Um die elektrochemische Reaktion zu detektieren ist ebenfalls in dem Gehäuse, bevorzugt in einem vom Innenraum separaten Elektronikraum, eine Messelektronik angeordnet. Die Messelektronik ist über Edelmetalldrähte mit den im Innenraum angeordneten Elektroden der elektrochemischen Zelle verbunden. Sie kann dazu ausgebildet sein, ein Messsignal kabellos, beispielsweise via Bluetooth oder eine vergleichbar kabellose Signalübertragung, an einen Empfänger zu senden, der außerhalb des Körpers des Individuums angeordnet ist, das den Sensor verschluckt hat.

Der Elektrolyteinlass ist eine Öffnung im Gehäuse des elektrochemischen Sensors. Die Öffnung verbindet das Innere des Gehäuses mit der äußeren Umgebung des elektrochemischen Sensors. Durch die Öffnung kann eine Flüssigkeit, bevorzugt Magensaft, in das Innere des Gehäuses einströmen.

Der hydrophile Dichtstoff ist beispielsweise ein Glasvlies, hydrophiles PTFE (Polytetrafluorethylen) funktionalisiertes PO (Polyolefin) oder ähnliches. Durch seine hydrophile Eigenschaft saugt er Magensaft aus der Umgebung des Sensors auf. Gleichzeitig verhindert er aber, dass größere Partikel in das Innere des Gehäuses gelangen. Der Dichtstoff sorgt insofern dafür, dass der Magensaft durch den Elektrolyteinlass hindurch in das Innere des Gehäuses transportiert wird.

Mit anderen Worten, die Erfindung betrifft einen schluckbaren elektrochemischen Sensor, der sich dadurch auszeichnet, dass sein Gehäuse Elektrolyteinlässe aufweist, durch die ein wässriger Elektrolyt, beispielsweise Magensäure, aus der Umgebung des Sensors in das Innere eindringen kann und dort als Elektrolyt dient. Der elektrochemische Sensor wird insofern durch das Verschlucken und das Eindringen der Magensäure in den betriebsbereiten Zustand überführt.

In einer ersten Ausführungsvariante ist vorgesehen, dass das Innere des Gehäuses mit einem hydrophilen Füllstoff ausgefüllt ist. Der hydrophile Füllstoff kann dann als Docht wirken, der den Magensaft aus der äußeren Umgebung des verschluckten Sensors gezielt zu den Elektroden transportiert. Der hydrophile Füllstoff kann aus dem gleichen Material bestehen, aus dem auch der hydrophile Dichtstoff besteht. Auf diese Weise kann sichergestellt werden, dass der als Elektrolyt fungierende Magensaft nicht nur in den Elektrolyteinlass hineingesaugt wird, sondern tatsächlich auch das Innere des Gehäuses füllt. Mit Hilfe eines solchen Dochts aus hydrophilem Füllmaterial kann außerdem sichergestellt werden, dass sich die für eine elektrochemische Reaktion zum Gasnachweis notwendige Dreiphasengrenze korrekt ausbildet. Der Docht, der aus dem hydrophilen Füllstoff gebildet ist und das Innere des Gehäuses ausfüllt, stellt mithin sicher, dass einerseits ausreichend aber auch nicht zu viel Elektrolyt von außen durch den Elektrolyteinlass bis zu den Elektroden transportiert wird. Der hydrophile Füllstoff ist dabei bevorzugt so ausgestaltet, dass der Transport des Elektrolyten sowohl durch die Hydrophilität des Materials als auch durch Kapillarkräfte beeinflusst wird. Die Stärke der wirkenden Kapillarkräfte kann beispielsweise durch die Wahl der Porengröße des Füllstoffs beeinflusst werden. Als besonders geeignet haben sich auch hier Glasfasersystem, bspw. Glasfaservliese, herausgestellt.

Außerdem ist vorstellbar, dass das Gehäuse mehrere Elektrolyteinlassöffnungen aufweist. Das bietet den Vorteil, dass der Magensaft mehrere Möglichkeiten für einen Eintritt in den Innenraum des Gehäuses hat. Auf diese Weise ist sichergestellt, dass auch im Falle der Blockade einer der Einlassöffnungen durch Speisepartikel oder ähnliches ausreichend Magensaft in den Innenraum des Gehäuses gelangen kann. Dabei ist auch vorstellbar, dass alle Öffnungen und die gesamte Kapsel mit hydrophilen Fasern gefüllt sind.

Man erkennt, dass es günstig ist, wenn der Sensor eine Kapsel ist. Unter einer Kapsel wird dabei allgemein ein zumeist kleiner, runder Behälter verstanden, der zum Transport von zu schützenden Gegenständen verwendbar ist. Ein solcher Behälter hat üblicherweise ein Gehäuse und in dessen Inneren der zu transportierende Gegenstand angeordnet ist. Dabei kann das Gehäuse der Kapsel das Gehäuse des elektrochemischen Sensors bilden, der übrige Aufbau des Sensors entspricht dem oben bereits ausgeführten und den nachfolgenden Ausführungen. Alternativ ist auch denkbar, dass der Sensor tablettenförmig ist. In jedem Fall kann der Sensor als gekapselter Sensor bezeichnet werden.

Denkbar ist, dass das Gehäuse aus inertem Kunststoff besteht. Beispielsweise kann das Gehäuse aus medizinisch verwendbarem Polypropylen oder Polyethylen hergestellt sein. Ein inerteres Material reagiert, wenn überhaupt, dann nur in verschwindend geringem Maß, bevorzugt überhaupt nicht, mit Reagenzien und Substanzen der Umgebung. Man erkennt, dass es insofern besonders günstig ist, wenn der inerte Kunststoff ein Kunststoff ist, der gegenüber Magensaft inert ist. Ein gegenüber Magensaft inerter Kunststoff insbesondere gegenüber Salzsäure und Verdauungsenzymen inert.

Der Sensor könnte außerdem auch ein weiches, inertes Material als Hülle aufweist. Eine solche Hülle kann beispielsweise als Überzug an der Außenseite des Gehäuses aufgebracht sein. Diese Hülle kann dazu dienen, die Schluckbarkeit des Sensors zu verbessern. Denkbar ist beispielsweise, dass die Hülle aus Silikon oder einem anderen medizinisch inerten Material besteht. Ein medizinisch inertes Material ist dabei ein Material, das keine Reaktion mit Körpersäften, beispielsweise Magensaft oder andere Verdauungssäften eingeht und insofern auch nicht im Körper resorbiert, sondern unverändert wieder ausgeschieden wird. Sofern die Hülle aus einem entsprechenden beständigen und inerten Material ausgebildet ist, ist sie selbstverständlich so ausgebildet, dass die Einlassöffnungen im Gehäuse, d.h. also Gaseinlass und Elektrolyteinlass nicht verschließt.

Alternativ könnte der Sensor auch eine Hülle aus einem weichen und resorbierbaren Material aufweisen. Ein solches weiches und resorbierbares Material könnte beispielsweise Gelatine sein. Auch mit einer solchen Hülle kann die Schluckbarkeit des Sensors noch verbessert werden. Das resorbierbare Material kann beispielsweise durch die Magensäfte aufgelöst werden. In diesem Fall ist es auch möglich, dass die Hülle die Einlassöffnungen im Gehäuse zunächst bedeckt. Dies kann beispielsweise günstig für den Herstellungsprozess sein. Der Sensor könnte dann einfach komplett in Gelatine getaucht werden.

In jedem Fall ist es sinnvoll, wenn der Gaseinlass unmittelbar vor der Arbeitselektrode liegt. Auf diese Weise kann die Strecke, die das Gas innerhalb des Sensors zurücklegen muss, bis es an der Arbeitselektrode umgesetzt wird, geringgehalten werden. Vorteilhaft ist dabei, wenn vor dem Gaseinlass vor der Arbeitselektrode ist eine hydrophobe Membran angeordnet ist. Eine solche Membran kann zum einen als physische Barriere dienen und das Eindringen von Speisepartikeln verhindern. Zum anderen kann die hydrophobe Membran das Eindringen von Magensaft durch den Gaseinlass verhindern. Die hydrophobe Membran kann engporig ausgebildet sein, um das Eindringen von anderen Materialien außer Gas zusätzlich zu erschweren. Unter einer engporigen Membran wird dabei eine Membran mit einer Porengröße von höchstens 5µm verstand. Günstig ist es dabei, wenn die Porengröße weniger als 5µm, bevorzugt weniger als 4µm, besonders bevorzugt weniger als 3µm, ganz besonders bevorzugt 2µm oder weniger aufweist.

Zusätzlich zu Arbeits- und Gegenelektrode kann der Sensor noch eine Referenzelektrode aufweisen. Die Referenzelektrode ist ebenfalls im Innenraum des Gehäuses angeordnet und kommt auf diese Weise ebenfalls in elektrisch leitenden Kontakt mit dem Elektrolyten. Sie kann zur Kalibrierung und Überprüfung der Funktionsfähigkeit des erfindungsgemäßen Sensors verwendet werden. Dies ist insbesondere deshalb von Vorteil, weil der verschluckte Sensor nicht ohne weiteres von außen zugänglich ist, um ihn zu kalibrieren oder zu warten.

In jedem Fall kann das Elektrodenmaterial ausgewählt sein aus Palladium, Platin, Rhodium, Iridium, Kohlenstoff und/oder Gold. Denkbar sind auch Gemische aus den zuvor genannten Materialien. Insbesondere Kohlenstoff und Gold bieten den zusätzlichen Vorteil, dass es sich dabei um inerte Materialien handelt. Auch die übrigen Elektrodenmaterialien können aufgrund der erfindungsgemäßen Bauweise des Sensors bedenkenlos verwendet werden, da sie sich ausschließlich im Inneren des Sensors befinden und mithin nicht in Kontakt mit Körpergewebe kommen können. Man erkennt, dass die Bauweise des Sensors als gekapselter Sensor diesbezüglich einen weiteren Vorteil bietet.

Dabei ist es auch denkbar, dass sind zwei Arbeitselektroden vorhanden sind. Dies ist beispielsweise dann von Vorteil, wenn mehrere unterschiedliche Gase nachgewiesen werden sollen. So kann beispielsweise die erste Arbeitselektrode aus Iridium sein. Eine solche Iridiumelektrode kann selektiv zum Nachweis von H₂S verwendet werden. Die zweite Arbeitselektrode kann aus Kohlenstoff sein. Eine Kohlenstoffelektrode kann dann zum selektiven Nachweis von NO verwendet werden. Enthält ein Sensor, der eine erste Arbeitselektrode aus Iridium und eine zweite Arbeitselektrode aus Kohlenstoff aufweist, kann mithin zum selektiven Nachweis von H₂S und NO verwendet werden. Dies ist beispielsweise hilfreich, wenn eine sich anbahnende Entzündungswelle im Verlauf einer chronischen Colitis nachgewiesen werden soll.

In einer Ausführungsvariante ist vorstellbar, dass der Sensor mit einem Elektrolyt befüllbar ist, wobei der Elektrolyt ausgewählt ist aus der Gruppe enthaltend Zitronensäure, Ameisensäure, Essigsäure, Salzsäure oder Phosphorsäure. Dabei kann der Sensor insbesondere vor dem Verschlucken mit dem Elektrolyt befüllbar sein. Zu diesem Zweck wird der bereitgestellte, elektrolytfreie Sensor in ein Elektrolytbad gelegt. Das Elektrolytbad besteht beispielsweise aus einer Lösung aus Zitronensäure, Ameisensäure, Essigsäure oder dergleichen. Besonders günstig ist es, wenn das Elektrolytbad aus einer Zitronensäurelösung besteht. Die Zitronensäure ist bevorzugt eine wässrige Lösung wobei die Zitronensäure in einer Konzentration im Bereich von 0,1 molar bis 2 molar, bevorzugt 0,5 molar bis 1 molar, besonders bevorzugt 0,75 molar vorliegt. In einer weiteren Ausführungsvariante ist es besonders günstig, wenn das Elektrolytbad aus einer Salzsäurelösung besteht. Die Salzsäurelösung ist eine wässrige Lösung, wobei die Salzsäure in einem Konzentrationsbereich von 0,1 molar bis 2 molar, bevorzugt von 0,5 molar bis 1 molar, ganz besonders bevorzugt 1 molar vorliegt. Es versteht sich von selbst, dass geringe Toleranzen des Konzentrationsbereichs ebenfalls zu den entsprechenden Ausführungsbeispielen gehören. In noch einer weiteren Ausführungsvariante ist es besonders günstig, wenn das Elektrolytbad aus einer Phosphorsäurelösung besteht. Die Phosphorsäurelösung ist eine wässrige Lösung, wobei die Phosphorsäure in einem Konzentrationsbereich von 0,1 molar bis 2 molar, bevorzugt von 0,5 molar bis 1 molar, ganz besonders bevorzugt 1 molar vorliegt. Es versteht sich von selbst, dass auch hier geringe Toleranzen des Konzentrationsbereichs ebenfalls zu den entsprechenden Ausführungsbeispielen gehören.

Man erkennt, dass im Sinne der vorliegenden Lösung auch ein Verfahren zur Bereitstellung eines schluckbaren elektrochemischen Sensors von Vorteil ist, wobei das Verfahren die Schritte aufweist:
a. Bereitstellen eines unbefüllten elektrochemischen Sensors
b. Bereitstellen einer Elektrolytlösung
c. Baden des unbefüllten elektrochemischen Sensors in der Elektrolytlösung
d. Bereitstellen des vorbefüllten elektrochemischen Sensors.

Optional kann der Sensor auch zwischen den Schritten c und d, d.h. also nachdem er durch das Bad in der Elektrolytlösung vorbefüllt wurde und bevor er zum Verschlucken bereitgestellt wird mit einem Coating versehen werden. Beispielsweise kann dabei das oben bereits beschriebene Coating mit Gelatine aufgebracht werden. Andere Coating-Materialien sind selbstverständlich auch denkbar. Insofern kann das Verfahren auch den folgenden Schritt e aufweisen:
e. Coating des vorbefüllten Sensors (10) mit einer bei Kontakt mit Magensäure selbstauflösenden Schicht.

Schritt e wird bevorzugt nach Schritt c aber vor Schritt d ausgeführt.

In jedem Fall ist es günstig, wenn das Vorbefüllen des elektrochemischen Sensors entsprechend Schritt c auch ein kurzes Abspülen des Sensors nach dem Baden des unbefüllten elektrochemischen Sensors einschließt. Das Abspülen kann beispielsweise durch kurzes Abspülen unter fließend Wasser unmittelbar vor dem Verschlucken erfolgen. Bevorzugt ist jedoch, den in der Elektrolytlösung gebadeten Sensor kurz in ein Abspülbad zu tauchen. Das Abspülbad kann beispielsweise destilliertes Wasser oder eine physiologische Kochsalzlösung enthalten.

Besonders günstig ist es, wenn das entsprechend Schritt b bereitgestellte Elektrolytbad einen Elektrolyten aufweist, der ausgewählt ist aus der Gruppe enthaltend Zitronensäure, Ameisensäure, Essigsäure, Salzsäure, Phosphorsäure, bevorzugt Zitronensäure, Ameisensäure, Salzsäure oder Phosphorsäure, ganz besonders bevorzugt Zitronensäure, Salzsäure oder Phosphorsäure.

Dabei ist beispielsweise denkbar, dass der unbefüllte Sensor zunächst einem medizinischen oder pharmazeutischen Personal zur Verfügung gestellt wird. Dieses führt dann die zur Vorbefüllung notwendigen Schritte und das Coating des Sensors aus. Dabei kann das Coating beispielsweise durch das Eintauchen des vorbefüllten Sensors in eine Gelatinelösung oder dergleichen erfolgen. Anschließend kann das Personal dann den so vorbereiteten Sensor an einen Patienten ausgeben, der diesen dann verschluckt.

Nach dem Verschlucken kann dann zusätzlich zu dem bereits in dem Sensor befindlichen Elektrolyten, wie bereits oben beschrieben, Magensäure durch einen oder mehrere der Elektrolyteinlässe in den Sensor einströmen.

In einer weiteren vorteilhaften Ausführungsvariante umfasst die Lösung der Aufgabe mithin auch ein Kit zum Nachweis von Intestinalen Gasen, wobei das Kit einen unbefüllten elektrochemischen Sensor entsprechend dem vorausgehend ausgeführten, sowie eine Elektrolytzubereitung aufweist.

Dabei ist es vorstellbar, dass die Elektrolytzubereitung als gebrauchsfertige flüssige Zubereitung, als konzentrierte flüssige Zubereitung oder als Pulver vorliegt. Mit Hilfe des Kits kann medizinisches oder pharmazeutisches Personal oder auch ein Patient selbst den schluckbaren elektrochemischen Sensor unmittelbar vor Gebrauch entsprechend vorbefüllen. Liegt die Elektrolytzubereitung als Pulver vor, so kann diese beispielsweise in einer entsprechend vorgegebenen Menge an Flüssigkeit aufgelöst werden. Beispielsweise in einer in der Gebrauchsanleitung vorgegebenen Menge Leitungswasser oder in destilliertem Wasser. Liegt die Elektrolytzubereitung als konzentrierte flüssige Zubereitung vor, so kann diese vor dem Baden des unbefüllten Sensors in der Elektrolytzubereitung entsprechend den Angaben in der Gebrauchsanweisung verdünnt werden, bis die gewünschte Gebrauchskonzentration erreicht ist.

Darüber hinaus kann eine solches Kit auch eine entsprechende Abspüllösung bereitstellen. Denkbar ist auch, dass das Kit entsprechende Gefäße umfasst, in denen die Elektrolytzubereitung und die Abspüllosung zum Eintauchen des Sensors bereitgestellt werden können.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:
- Fig. 1: einen schematischen Querschnitt durch einen erfindungsgemäßen Sensor
- Fig. 2: einen weiteren schematischen Querschnitt durch einen erfindungsgemäßen Sensor
- Fig. 3: eine schematische Übersicht über ein Verfahren zur Bereitstellung eines schluckbaren elektrochemischen Sensors

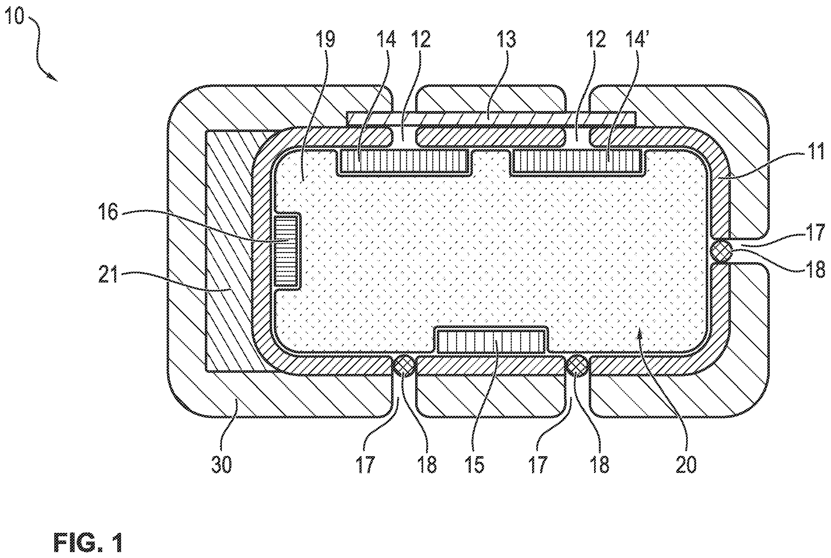

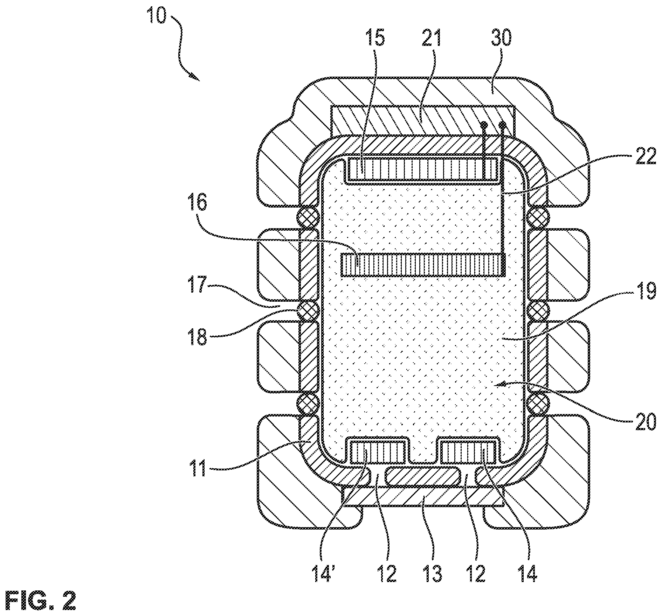

Sowohl in Fig.1 als auch in Fig. 2 erkennt man einen Querschnitt durch einen Gassensor 10. Der Gassensor 10 hat ein Gehäuse 11. Das Gehäuse 11 hat einen Innenraum 20. In dem Innenraum 20 sind eine erste Arbeitselektrode 14 und eine zweite Arbeitselektrode 14' angeordnet. Vorstellbar sind auch Ausführungsformen, die nur eine Arbeitselektrode 14 aufweisen. Weiterhin ist in dem Innenraum 20 eine Gegenelektrode 15 angeordnet, sowie eine Referenzelektrode 16.

An der Außenseite des Gehäuses 11 ist eine Elektronikeinheit 21 angeordnet. Die Elektronikeinheit 21 ist in einer dichten Kapsel verbaut. Die Elektroden 14, 14', 15, 16 stehen über Drahtverbindungen 22, die aus der Elektronikeinheit 21 heraus und durch das Gehäuse 11 hindurchgeführt sind, mit der Elektronikeinheit 21 in Verbindung, wie insbesondere in Fig. 2 zu erkennen ist. Diese Drahtverbindung ist nicht für alle Elektroden in den Fig. 1 und 2 zu sehen, weil sie teilweise außerhalb der Querschnittsebene der Darstellung liegt. Selbstverständlich ist aber für jede der Elektroden eine entsprechende Drahtverbindung 22 vorhanden.

Man erkennt, dass das Gehäuse 11 mehrere Öffnungen aufweist, nämlich Öffnungen, die als Gaseinlass 12 dienen, und Öffnungen, die als Elektrolyteinlass 17 dienen.

Der Gaseinlass 12 ist jeweils so angeordnet, dass auf der Innenseite des Gehäuses 11, direkt hinter dem Gaseinlass 12 eine Arbeitselektrode 14, 14' liegt. Außerdem wird jeder Gaseinlass 12 von einer hydrophoben Membran 13 verschlossen. Auf diese Weise kann durch den Gaseinlass 12 ausschließlich Gas in den Sensor eindringen und zur Arbeitselektrode 14, 14' gelangen. Wässrige Flüssigkeiten werden aufgrund der hydrophoben Eigenschaft der Membran abgewiesen. Feste Partikel, bspw. Speisepartikel, werden durch physische Barrierewirkung der Membran am Eindringen gehindert. Im gezeigten Beispiel der Figuren 1 und 2 liegt die hydrophobe Membran 13 an der Außenseite des Gehäuses 12. Denkbar ist auch, dass sie an der Innenseite des Gehäuses 12 angeordnet ist, wobei jedoch die in den Figuren dargestellte Ausführungsform bevorzugt wird. Weiterhin erkennt man, dass in den Figuren 1 und 2 eine hydrophobe Membran 13 alle Gaseinlässe 12 bedeckt. Denkbar ist dabei selbstverständlich auch, dass jeder Gaseinlass 12 mit einer eigenen hydrophoben Membran 13 verschlossen ist, wobei auch hier die in den Figuren dargestellte Ausführungsvariante bevorzugt ist.

Die Öffnungen, die als Elektrolyteinlass 17 dienen, sind jeweils hydrophile Dichtungen 18 angeordnet. Diese leiten aufgrund ihrer hydrophilen Eigenschaften wässrige Flüssigkeiten in den Innenraum 20 des Gehäuses 11. Die hydrophilen Dichtungen 18 verhindern jedoch ebenso wie die hydrophobe Membran 13, dass feste Partikel in den Innenraum 20 gelangen können.

Der Innenraum 20 des Gehäuses 11 ist weiterhin mit einem hydrophilen Füllstoff 19 ausgefüllt. Dieser kann wie ein Schwamm oder Docht wirken und beispielsweise das Eindringen der als Elektrolyt dienenden Magensäure auf diese Weise unterstützen. Weiterhin kann der hydrophile Füllstoff 19 den erfolgreich eingedrungenen Elektrolyten im Innenraum 20 des Gehäuses 11 halten.

Man erkennt weiter in den Figuren 1 und 2, dass die gesamte Vorrichtung 10 von einer Hülle 30 umgeben ist. Die Hülle 30 bildet eine Kapsel um den Sensor 10. Die Hülle 30 besteht beispielsweise aus einem weichen, inerten Material, wie oben beschrieben. Man erkennt, dass die Hülle 30 auch die hydrophobe Membran umschließt. Lediglich an den Stellen, an denen die Gaseinlässe 12 und die Elektrolyteinlässe 17 ausgebildet sind, weist die Hülle 30 ebenfalls Durchlassöffnungen auf.

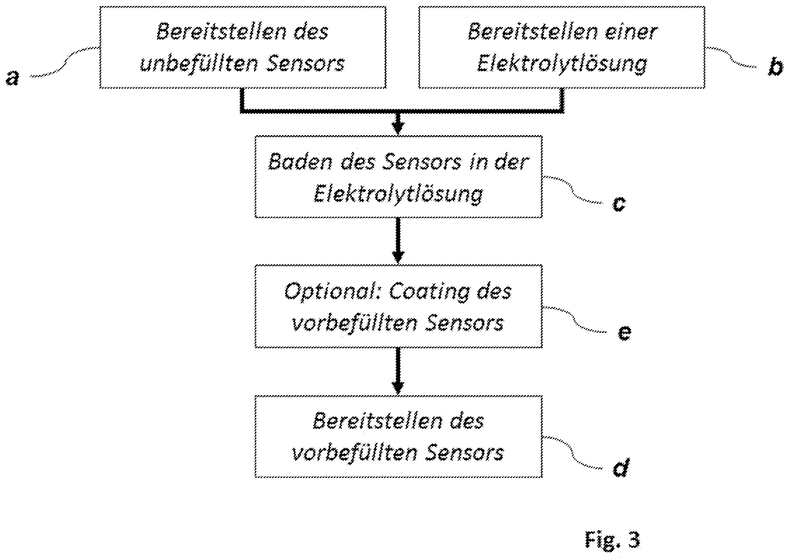

In Fig. 3 erkennt man, dass ein entsprechender schluckbarer elektrochemischer Sensor bereitgestellt werden kann, in dem in einem ersten Schritt a ein unbefüllter Sensor 10 bereitgestellt wird. Dieser unbefüllte Sensor 10 entspricht dem oben beschriebenen und in den Figuren 1 und 2 dargestellten Sensor 10. Um Wiederholungen zu vermeiden, wird insofern für alle Merkmale des bereitgestellten unbefüllten Sensors 10 auf das oben ausgeführte verwiesen. In einem weiteren Schritt b wird eine Elektrolytlösung bereitgestellt. Diese kann beispielsweise Zitronensäure, Ameisensäure oder Essigsäure als Elektrolyt aufweisen. Entsprechend Schritt c des Verfahrens wird der bereitgestellte unbefüllte Sensor 10 dann in der bereitgestellten Elektrolytlösung gebadet. Dabei wird der Sensor mit dem entsprechenden Elektrolyt vorbefüllt. Entsprechend Schritt e kann der so vorbefüllte Sensor 10 bei Bedarf optional mit einem Coating versehen werden, bevor er in jedem Fall entsprechend Schritt d zur weiteren Verwendung bereitgestellt wird.

In einem bevorzugten Ausführungsbeispiel ist die entsprechend Schritt b bereitgestellte Elektrolytlösung eine Lösung aus 0,75 molarer Zitronensäure in Wasser.

In einem weiteren Ausführungsbeispiel ist die entsprechend Schritt b bereitgestellte Elektrolytlösung eine wässrige Lösung aus 1 molarer Salzsäure.

In noch einem weiteren Ausführungsbeispiel ist die entsprechend Schritt b bereitgestellte Elektrolytlösung eine wässrige Lösung aus 1 molarer Phosphorsäure.

Die Erfindung ist nicht auf eine der vorbeschriebenen Ausführungsformen beschränkt, sondern in vielfältiger Weise abwandelbar.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritte, können sowohl für sich als auch in den verschiedensten Kombinationen erfindungswesentlich sein.

In jedem Fall ist bei einem elektrochemischer Sensor 10 mit einem Gehäuse 11, mit wenigstens einer Arbeitselektrode 14 und mit einer Gegenelektrode 15, wobei das Gehäuse 11 einen Innenraum 20 aufweist, in dem die Arbeitselektrode 14 und die Gegenelektrode 15 angeordnet sind und wobei das Gehäuse 11 wenigstens einen Gaseinlass 12 hat, wobei jeder Gaseinlass 12 mit einer gasdurchlässigen Membran 13 verschlossen ist, vorgesehen, dass das Gehäuse 13 wenigstens einen Elektrolyteinlass 17 hat, wobei jeder Elektrolyteinlass 17 mit einem hydrophilen Dichtstoff 18 ausgefüllt ist. Dabei ist es günstig, wenn der Innenraum 20 des Gehäuses 20 mit einem hydrophilen Füllstoff 19 ausgefüllt ist und/oder wenn das Gehäuse 20 mehrere Elektrolyteinlassöffnungen 17 aufweist. Vorteilhaft ist auch, wenn der Sensor 10 ist eine Kapsel ist, wenn das Gehäuse 20 aus inertem Kunststoff besteht und/oder wenn der Sensor 10 ein weiches, inertes Material als Hülle 30 aufweist. Zweckmäßigerweise liegt dabei der Gaseinlass 12 unmittelbar vor der Arbeitselektrode 14, wobei vor dem Gaseinlass 12 eine hydrophobe Membran 13 angeordnet ist.

Weiterhin ist es günstig, wenn der Sensor 10 eine Referenzelektrode 16 aufweist. Dabei kann das Elektrodenmaterial ausgewählt sein aus Palladium, Platin, Rhodium, Iridium, Kohlenstoff und/oder Gold. Bevorzugt sind zwei Arbeitselektroden 14, 14' vorhanden. Dabei kann die erste Arbeitselektrode 14 aus Iridium sein. Die zweite Arbeitselektrode 14' kann aus Kohlenstoff sein.

### Bezugszeichenliste

- 10: Gassensor
- 11: Gehäuse
- 12: Gaseinlass
- 13: hydrophobe Membran
- 14: Arbeitselektrode
- 14': Arbeitselektrode
- 15: Gegenelektrode
- 16: Referenzelektrode
- 17: Elektrolyteinlass
- 18: hydrophiler Dichtstoff
- 19: hydrophiler Füllstoff
- 20: Innenraum
- 21: Elektronikeinheit
- 22: Drahtverbindung
- 30: Hülle

## Patentansprüche

1. Schluckbarer Elektrochemischer Sensor (10) zum Nachweis von Gasen im Intestinaltrakt eines Menschen mit einem Gehäuse (11), mit wenigstens einer Arbeitselektrode (14) und mit einer Gegenelektrode (15), wobei das Gehäuse (11) einen Innenraum (20) aufweist, in dem die Arbeitselektrode (14) und die Gegenelektrode (15) angeordnet sind und wobei das Gehäuse (11) wenigstens einen Gaseinlass (12) hat, wobei jeder Gaseinlass (12) mit einer gasdurchlässigen Membran (13) verschlossen ist, **dadurch gekennzeichnet, dass** das Gehäuse (11) wenigstens einen Elektrolyteinlass (17) hat, wobei jeder Elektrolyteinlass (17) mit einem hydrophilen Dichtstoff (18) ausgefüllt ist.

2. Elektrochemischer Sensor nach Anspruch 1, **dadurch gekennzeichnet, dass** der Innenraum (20) des Gehäuses (11) mit einem hydrophilen Füllstoff (19) ausgefüllt ist.

3. Elektrochemischer Sensor nach einem der vorhergehenden Ansprüche, **dadurch gekennzeichnet, dass** das Gehäuse (11) mehrere Elektrolyteinlassöffnungen (17) aufweist.

4. Elektrochemischer Sensor nach einem der vorhergehenden Ansprüche, **dadurch gekennzeichnet, dass** der Sensor (10) eine Kapsel ist.

5. Elektrochemischer Sensor nach einem der vorhergehenden Ansprüche, **dadurch gekennzeichnet, dass** das Gehäuse (11) aus inertem Kunststoff besteht.

6. Elektrochemischer Sensor nach einem der vorhergehenden Ansprüche, **dadurch gekennzeichnet, dass** der Sensor (10) ein weiches, inertes Material als Hülle (30) aufweist.

7. Elektrochemischer Sensor nach einem der vorhergehenden Ansprüche, **dadurch gekennzeichnet, dass** der Gaseinlass (12) unmittelbar vor der Arbeitselektrode (14) liegt.

8. Elektrochemischer Sensor nach einem der vorhergehenden Ansprüche, **dadurch gekennzeichnet, dass** vor dem Gaseinlass (12) vor der Arbeitselektrode (14) eine hydrophobe Membran (13) angeordnet ist.

9. Elektrochemischer Sensor nach einem der vorhergehenden Ansprüche, **dadurch gekennzeichnet, dass** zwei Arbeitselektroden (14, 14') vorhanden sind.

10. Elektrochemischer Sensor nach einem der vorhergehenden Ansprüche, **dadurch gekennzeichnet, dass** der Sensor mit einem Elektrolyt befüllbar ist, wobei der Elektrolyt ausgewählt ist aus der Gruppe enthaltend Zitronensäure, Ameisensäure, Essigsäure, Salzsäure, Phosphorsäure.

11. Verfahren zur Bereitstellung eines schluckbaren elektrochemischen Sensors entsprechend einem der Ansprüche 1 bis 10, **dadurch gekennzeichnet, dass** das Verfahren die Schritte aufweist:
a. Bereitstellen eines unbefüllten elektrochemischen Sensors (10);
b. Bereitstellen einer Elektrolytlösung;
c. Baden des unbefüllten elektrochemischen Sensors (10) in der Elektrolytlösung;
d. Bereitstellen des vorbefüllten elektrochemischen Sensors (10).

12. Verfahren nach Anspruch 11, **dadurch gekennzeichnet, dass** das Verfahren außerdem den Schritt aufweist:
e. Coating des vorbefüllten Sensors (10) mit einer bei Kontakt mit Magensäure selbstauflösenden Schicht.

13. Verfahren nach einem der Ansprüche 11 oder 12, **dadurch gekennzeichnet, dass** das entsprechend Schritt b bereitgestellte Elektrolytbad einen Elektrolyten aufweist, der ausgewählt ist aus der Gruppe enthaltend Zitronensäure, Ameisensäure, Essigsäure, Salzsäure, Phosphorsäure, bevorzugt Zitronensäure, Ameisensäure, Salzsäure oder Phosphorsäure, ganz besonders bevorzugt Zitronensäure, Salzsäure oder Phosphorsäure.

14. Kit zum Nachweis von Intestinalen Gasen, wobei das Kit einen unbefüllten elektrochemischen Sensor entsprechend wenigstens einem der Ansprüche 1 bis 10, sowie eine Elektrolytzubereitung aufweist.

15. Kit nach Anspruch 14, **dadurch gekennzeichnet, dass** die Elektrolytzubereitung als gebrauchsfertige flüssige Zubereitung, als konzentrierte flüssige Zubereitung oder als Pulver vorliegt.

## Claims

1. Ingestible electrochemical sensor (10) for detecting gases in the intestinal tract of a human, comprising a housing (11), at least one working electrode (14) and a counter electrode (15), the housing (11) comprising an interior space (20) in which the working electrode (14) and the counter electrode (15) are arranged, and the housing (11) having at least one gas inlet (12), each gas inlet (12) being closed by a gas-permeable membrane (13), **characterized in that** the housing (11) has at least one electrolyte inlet (17), each electrolyte inlet (17) being filled with a hydrophilic sealant (18).

2. Electrochemical sensor according to claim 1, **characterized in that** the interior (20) of the housing (11) is filled with a hydrophilic filler (19).

3. Electrochemical sensor according to either of the preceding claims,
**characterized in that** the housing (11) comprises a plurality of electrolyte inlet openings (17).

4. Electrochemical sensor according to any of the preceding claims,
**characterized in that** the sensor (10) is a capsule.

5. Electrochemical sensor according to any of the preceding claims,
**characterized in that** the housing (11) is made of inert plastics material.

6. Electrochemical sensor according to any of the preceding claims,
**characterized in that** the sensor (10) comprises a soft, inert material as a casing (30).

7. Electrochemical sensor according to any of the preceding claims,
**characterized in that** the gas inlet (12) is located directly in front of the working electrode (14).

8. Electrochemical sensor according to any of the preceding claims,
**characterized in that** a hydrophobic membrane (13) is arranged in front of the gas inlet (12) in front of the working electrode (14).

9. Electrochemical sensor according to any of the preceding claims,
**characterized in that** two working electrodes (14, 14') are present.

10. Electrochemical sensor according to any of the preceding claims,
**characterized in that** the sensor can be filled with an electrolyte, the electrolyte being selected from the group containing citric acid, formic acid, acetic acid, hydrochloric acid, phosphoric acid.

11. Method for providing an ingestible electrochemical sensor according to any of claims 1 to 10, **characterized in that** the method comprises the steps of:
a. providing an unfilled electrochemical sensor (10);
b. providing an electrolyte solution;
c. bathing the unfilled electrochemical sensor (10) in the electrolyte solution;
d. providing the prefilled electrochemical sensor (10).

12. Method according to claim 11, **characterized in that** the method also comprises the step of
e. coating the prefilled sensor (10) with a layer that dissolves upon contact with gastric acid.

13. Method according to either of claims 11 or 12, **characterized in that** the electrolyte bath provided according to step b comprises an electrolyte which is selected from the group containing citric acid, formic acid, acetic acid, hydrochloric acid, phosphoric acid, preferably citric acid, formic acid, hydrochloric acid or phosphoric acid, particularly preferably citric acid, hydrochloric acid or phosphoric acid.

14. Kit for detecting intestinal gases, wherein the kit comprises an unfilled electrochemical sensor according to at least one of claims 1 to 10, and an electrolyte preparation.

15. Kit according to claim 14, **characterized in that** the electrolyte preparation is available as a ready-to-use liquid preparation, as a concentrated liquid preparation or as a powder.

## Revendications

1. Capteur électrochimique (10) pouvant être avalé pour la détection de gaz dans le tractus intestinal d'un être humain, comportant un boîtier (11), comportant au moins une électrode de travail (14) et comportant une contre-électrode (15), dans lequel le boîtier (11) présente un espace intérieur (20) dans lequel sont disposées l'électrode de travail (14) et la contre-électrode (15) et dans lequel le boîtier (11) possède au moins une entrée de gaz (12), dans lequel chaque entrée de gaz (12) est fermée par une membrane (13) perméable aux gaz,
**caractérisé en ce que** le boîtier (11) possède au moins une entrée d'électrolyte (17), dans lequel chaque entrée d'électrolyte (17) est remplie d'un matériau d'étanchéité hydrophile (18).

2. Capteur électrochimique selon la revendication 1, **caractérisé en ce que** l'espace intérieur (20) du boîtier (11) est rempli d'une matière de charge hydrophile (19).

3. Capteur électrochimique selon l'une des revendications précédentes, **caractérisé en ce que** le boîtier (11) présente plusieurs orifices d'entrée d'électrolyte (17).

4. Capteur électrochimique selon l'une des revendications précédentes, **caractérisé en ce que** le capteur (10) est une capsule.

5. Capteur électrochimique selon l'une des revendications précédentes, **caractérisé en ce que** le boîtier (11) est en matière plastique inerte.

6. Capteur électrochimique selon l'une des revendications précédentes, **caractérisé en ce que** le capteur (10) présente un matériau souple et inerte comme enveloppe (30).

7. Capteur électrochimique selon l'une des revendications précédentes, **caractérisé en ce que** l'entrée de gaz (12) se situe immédiatement avant l'électrode de travail (14).

8. Capteur électrochimique selon l'une des revendications précédentes, **caractérisé en ce qu'**une membrane (13) hydrophobe est disposée en amont de l'entrée de gaz (12) avant l'électrode de travail (14).

9. Capteur électrochimique selon l'une des revendications précédentes, **caractérisé en ce que** deux électrodes de travail (14, 14') sont prévues.

10. Capteur électrochimique selon l'une des revendications précédentes, **caractérisé en ce que** le capteur peut être rempli d'un électrolyte, dans lequel l'électrolyte est choisi dans le groupe contenant acide citrique, acide formique, acide acétique, acide chlorhydrique, acide phosphorique.

11. Procédé permettant de fournir un capteur électrochimique pouvant être avalé conformément à l'une des revendications 1 à 10, **caractérisé en ce que** le procédé présente les étapes consistant à :
a. fournir un capteur électrochimique (10) non rempli ;
b. fournir une solution d'électrolyte ;
c. baigner le capteur électrochimique (10) non rempli dans la solution d'électrolyte ;
d. fournir le capteur électrochimique (10) prérempli.

12. Procédé selon la revendication 11, **caractérisé en ce que** le procédé en outre l'étape consistant à
e. revêtir le capteur (10) prérempli d'une couche autodissolvante au contact de l'acide gastrique.

13. Procédé selon l'une des revendications 11 ou 12, **caractérisé en ce que** le bain d'électrolyte fourni conformément à l'étape b présente un électrolyte qui est choisi dans le groupe contenant acide citrique, acide formique, acide acétique, acide chlorhydrique, acide phosphorique, de préférence acide citrique, acide formique, acide chlorhydrique ou acide phosphorique, de manière particulièrement préférée acide citrique, acide chlorhydrique ou acide phosphorique.

14. Kit pour la détection de gaz intestinaux, dans lequel le kit présente un capteur électrochimique non rempli conformément à au moins l'une des revendications 1 à 10, ainsi qu'une préparation d'électrolyte.

15. Kit selon la revendication 14, **caractérisé en ce que** la préparation d'électrolyte se présente sous la forme d'une préparation liquide prête à l'emploi, d'une préparation liquide concentrée ou d'une poudre.
